(19) Europäisches Patentamt / European Patent Office / Office européen des brevets

(11) **EP 4 545 958 A1**

(12) **EUROPEAN PATENT APPLICATION**
published in accordance with Art. 153(4) EPC

(43) Date of publication:
30.04.2025  Bulletin 2025/18

(21) Application number: 23826813.0

(22) Date of filing: 28.04.2023

(51) International Patent Classification (IPC):
*G01N 27/00* (2006.01)    *G01N 33/483* (2006.01)
*G01N 33/543* (2006.01)

(52) Cooperative Patent Classification (CPC):
G01N 27/00; G01N 33/483; G01N 33/543

(86) International application number:
PCT/JP2023/016945

(87) International publication number:
WO 2023/248624 (28.12.2023 Gazette 2023/52)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  24.06.2022   JP 2022101996

(71) Applicant: Aipore Inc.
Tokyo, 150-8512 (JP)

(72) Inventors:
• **NAONO, Norihiko**
  **Tokyo 150-8512 (JP)**
• **SAKAMOTO, Osamu**
  **Tokyo 150-8512 (JP)**
• **TAKEI, Hiroyasu**
  **Tokyo 150-8512 (JP)**

(74) Representative: **Mewburn Ellis LLP**
**Aurora Building**
**Counterslip**
**Bristol BS1 6BX (GB)**

(54) **METHOD, DEVICE, AND PROGRAM FOR DETECTING AND QUANTIFYING PROTEIN**

(57)    A method for estimating presence or absence or a concentration of an antigen or an antibody to be detected in a biological sample collected from a living body, including the steps of:
filtering the biological sample through a filter having a blocking size m to prepare a filtered sample;
preparing a measurement target sample by mixing:
antibody-modified particles having a particle diameter d and an antibody that binds to the antigen attached to its surface, or antigen-modified particles having a particle diameter d and an antigen that binds to the antibody attached to its surface,
the filtered sample, and
a first electrolyte solution;

to a sensor having a structure in which two chambers separated by a partition wall having a pore with a pore diameter D communicate with each other through the pore, and an electrode is provided in each of the two chambers, filling one of the two chambers of the sensor with the measurement target sample;

filling the other of the two chambers of the sensor with a second electrolyte solution to electrically connect the two chambers through the pores;
applying a voltage between two electrodes in each of the two chambers to allow an ionic current to flow between the two electrodes via the pore, and measuring a transient change in the ionic current that occurs each time the antibody-modified particle or the antigen-modified particle passes through the pore as a pulse waveform group consisting of a plurality of pulse waveforms; and
estimating the presence or absence or the concentration of the antigen or the antibody to be detected in the biological sample by analyzing the pulse waveform group;
wherein the blocking size m is equal to or more than a lower limit determined according to a size of the antigen or the antibody to be detected and is equal to or less than 1/2 of the pore diameter D, and
wherein the diameter d of the antibody-modified particle or the antigen-modified particle is 1/5 or more of the pore diameter D and less than the pore diameter D.

EP 4 545 958 A1

START

A target antibody that specifically binds to a target antigen is attached to the surface of a detection particle to prepare an antibody-modified particle. ⟶ S301

A biological sample with a known concentration of a target antigen is pretreated to prepare a known measurement target sample (described in FIG. 11). ⟶ S302

The known measurement target sample is filled in one chamber of the sensor and a second electrolyte is filled in the other chamber. ⟶ S303

A voltage is applied between the electrodes of the sensor, and the transient change in current when a particle passes through is measured as a pulse waveform. ⟶ S304

The correlation between the presence or absence and concentration of a known target antigen and the shape and distribution of the pulse signal of a known measurement target sample is modeled (trained in the case of AI) to determine the agglutination-accelerating antigen concentration. ⟶ S305

END

(A) Modeling with known samples (training in the case of AI)

FIG. 3A

START

A target antibody that specifically binds to a target antigen is attached to the surface of a detection particle to prepare an antibody-modified particle. — S311

A biological sample with a known concentration of a target antigen is pretreated to prepare a known measurement target sample (described in FIG. 11). — S312

The measurement target sample is filled in one chamber of the sensor and a second electrolyte is filled in the other chamber. — S313

A voltage is applied between the electrodes of the sensor, and the transient change in current when a particle passes through is measured as a pulse waveform. — S314

The presence or absence and concentration of the target antigen in a test sample is estimated from the shape of the pulse signal, its distribution, and a correlation model (in the case of AI, a trained AI model). — S315

END

(B) Detection or quantification of unknown samples (in the case of AI, estimation by trained AI)

FIG. 3B

## Description

### FIELD OF THE INVENTION

The present invention relates to a method, an apparatus, and a program for detecting and quantifying a protein contained in a sample.

### BACKGROUND OF THE INVENTION

Since proteins are the basic components of living organisms, their detection and quantification are fundamental tools in biological research. In addition, detection and quantification of various proteins are widely used in clinical testing for various diseases. In particular, if it were possible to easily, cheaply, and frequently test for trace markers of various diseases such as malignant tumors, neurological diseases, and collagen diseases, it would be very effective in preventing the disease from worsening through early detection and improving the quality of life of patients.

For the detection and quantification of trace proteins, ELISA (Enzyme-Linked Immuno Sorbent Assay), CLEIA (Chemiluminescent Enzyme Immunoassay), CLIA (Chemiluminescent Immunoassay), and the like, which label antibodies or antigens with chemiluminescent substances and measure absorbance or luminescence, are widely used.

A highly sensitive immunoassay technique, such as a so-called digital ELISA, has been proposed that improves sensitivity by developing the method of using such chemiluminescent substances and combining nano-sized wells with antibody-modified beads (Patent Literature 1). However, these methods require complicated measurement protocols and large, precise optical measurement systems, which require expensive measurement equipment, and therefore have not been widely used as a low-cost, simple, and frequently performed clinical test.

The problems with the above-mentioned conventional techniques are the complexity and time required for the measurement procedure. For example, in the case of ELISA, it is necessary to repeat washing after each treatment such as antibody immobilization, blocking, incubation, and the like. Adsorption during immobilization of the primary antibody, adsorption of the target protein to substances other than the antibody, adsorption of the detection antibody to substances other than the antigen, and residual antibody/antigen on the substrate all become noise when measuring absorbance and have a significant adverse effect on the sensitivity, specificity, and linearity of the detection and quantification of the target antigen. To prevent this, repeated cleaning is essential, which hinders shortening the inspection time and reducing costs.

On the other hand, a technique has been proposed for directly observing individual proteins without using nano-sized wells and optical systems (Non-Patent Literature 1). Here, the so-called pore electrical resistance method (Patent Literature 2) is used, in which nano-sized particles in an electrolyte are driven by electrophoresis and the transient change in electrical resistance is observed as they pass through pores close to the size of the particles. However, biological samples contain a large number of proteins, cells and their fragments, and in some cases pathogens such as viruses and bacteria. For this reason, the pulse waveform measured when the target antigen passes through the pore will mostly be that due to fine particles that are not the target of measurement. In the case of the above-mentioned ELISA, the problem of noise is avoided by utilizing specific binding to immobilized antigens or antibodies and washing away all other microparticles that are a source of noise. In addition, in the above-mentioned pore electrical resistance method, an attempt has been reported to remove fine particles that are a source of noise from the data to be analyzed by performing probabilistic calculations for each pulse waveform in data processing after measurement (Patent Literature 3).

### PRIOR ART

Patent Literature

   [Patent Literature 1] U.S. Patent No. 8,236,574

   [Patent Literature 1] U.S. Patent No. 9,726,636

   [Patent Literature 1] Japanese Patent No. 6,985,687

Non-Patent Literature

[Non-Patent Literature 1] Yusko, Erik C., et al. "Real-time shape approximation and fingerprinting of single proteins using a nanopore." Nature nanotechnology 12.4 (2017): 360-367.

### SUMMARY OF THE INVENTION

However, in the detection of proteins by the pore electrical resistance method, even if filtration is performed using a filter, other proteins and contaminating microparticles of a similar size to the target protein will pass through the filter, so there is no way to selectively select only the target protein as the one to pass through the pore in the pore electrical resistance method. In addition, the pulse waveform generated by the passage of a protein through a pore is extremely small, making it difficult to separate this from noise pulses by data processing after measurement.

Whether it is a conventional technique using immunoassays such as ELISA and chemiluminescence, or a technique using pore electrical resistance, the success or failure of practically meaningful high sensitivity in protein detection or quantification depends on noise signals originating from sources other than the protein being detected or quantified. Conventional techniques have not been able to efficiently remove noise signals easily and at low cost.

The present invention has been made in consideration of these circumstances, and realizes highly sensitive, inexpensive, rapid and simple detection and quantification of proteins (or antibodies or antigens) required for clinical tests involving a large amount of contaminants, without completely removing the contaminants. That is, the present invention can provide the following aspects.

[Aspect 1]

A method for estimating presence or absence or a concentration of an antigen or an antibody to be detected in a biological sample collected from a living body, comprising the steps of:

filtering the biological sample through a filter having a blocking size m to prepare a filtered sample;

preparing a measurement target sample by mixing:

antibody-modified particles having a particle diameter d and an antibody that binds to the antigen attached to its surface, or antigen-modified particles having a particle diameter d and an antigen that binds to the antibody attached to its surface,

the filtered sample, and

a first electrolyte solution;

to a sensor having a structure in which two chambers separated by a partition wall having a pore with a pore diameter D communicate with each other through the pore, and an electrode is provided in each of the two chambers, filling one of the two chambers of the sensor with the measurement target sample;

filling the other of the two chambers of the sensor with a second electrolyte solution to electrically connect the two chambers through the pores;

applying a voltage between two electrodes in each of the two chambers to allow an ionic current to flow between the two electrodes via the pore, and measuring a transient change in the ionic current that occurs each time the antibody-modified particle or the antigen-modified particle passes through the pore as a pulse waveform group consisting of a plurality of pulse waveforms; and

estimating the presence or absence or the concentration of the antigen or the antibody to be detected in the biological sample by analyzing the pulse waveform group;

wherein the blocking size m is equal to or more than a lower limit determined according to a size of the antigen or the antibody to be detected and is equal to or less than 1/2 of the pore diameter D, and

wherein the diameter d of the antibody-modified particle or the antigen-modified particle is 1/5 or more of the pore diameter D and less than the pore diameter D.

[Aspect 2]

The method according to aspect 1, wherein the blocking size m is 2 nm or more and 1/10 or less of the pore diameter D, and
a diameter $d_1$ of the antibody-modified particle or the antigen-modified particle is 1/5 or more of the pore diameter D and less than the pore diameter D.

[Aspect 3]

The method according to aspect 1 or 2, wherein an aggregation state of the antibody-modified particles or the antigen-modified particles in the biological sample is measured by measuring the transient change in the ionic current as a group of pulse waveforms consisting of a plurality of pulse waveforms.

[Aspect 4]

The method according to aspect 1, further comprising:

extracting a selected pulse waveform having a peak current equal to or more than an exclusion threshold from pulse waveform group; and

detecting the presence or absence of the antigen or the antibody to be detected in the biological sample or calculating an concentration estimate value thereof by analyzing the selected pulse waveform group;

wherein the diameter d of the antibody-modified particle or the antigen-modified particle is less than the pore diameter D, and the blocking size m is 1/2 or less of the diameter d.

[Aspect 5]

The method according to aspect 4, further comprising:
recursively determining the exclusion threshold value so that the concentration estimate value is close to the true value of the antigen or the antibody to be detected contained in the biological sample.

[Aspect 6]

The method according to any one of aspects 1 to 5, wherein the first electrolyte solution and the second electrolyte solution have different compositions.

[Aspect 7]

The method according to any one of aspects 1 to 5, wherein the first electrolyte solution and the second electrolyte solution have the same composition.

[Aspect 8]

A device for estimating presence or absence or a concentration of an antigen or an antibody to be detected in a biological sample collected from a living body, comprising:

a filter having a blocking size m;

a sensor having a structure in which two chambers separated by a partition wall having a pore with a pore diameter D communicate with each other through the pore, and an electrode is provided in each of the two chambers;

an interface for connecting to a computer via a network;

wherein the device is configured to:

prepare a measurement target sample by mixing:

antibody-modified particles having a particle diameter d and an antibody that binds to the antigen attached to its surface, or antigen-modified particles having a particle diameter d and an antigen that binds to the antibody attached to its surface,

the filtered sample, and

a first electrolyte solution;

fill one of the two chambers of the sensor with the measurement target sample;

fill the other of the two chambers of the sensor with a second electrolyte solution to electrically connect the two chambers through the pore; and

apply a voltage between two electrodes in each of the two chambers to allow an ionic current to flow between the two electrodes via the pore, and measure a transient change in the ionic current that occurs each time the antibody-modified particle or the antigen-modified particle passes through the pore as a pulse waveform group consisting of a plurality of pulse waveforms to obtain a data, and transmit the data to the computer via the interface;

wherein the blocking size m is equal to or more than a lower limit determined according to a size of the antigen or the antibody to be detected and is equal to or less than 1/2 of the pore diameter D, and the diameter d of the antibody-modified particle or the antigen-modified particle is 1/5 or more of the pore diameter D and less than the pore diameter D.

[Aspect 9]

The device according to aspect 8, wherein the blocking size m is equal to or less than 1/10 of the pore diameter D.

[Aspect 10]

A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform:

a step of filtering a biological sample through a filter having a blocking size m to prepare a filtered sample;

a step of prepare a measurement target sample by mixing:

antibody-modified particles having a particle diameter d and an antibody that binds to the antigen attached to its surface, or antigen-modified particles having a particle diameter d and an antigen that binds to the antibody attached to its surface,

the filtered sample, and

a first electrolyte solution;

to a sensor having a structure in which two chambers separated by a partition wall having a pore with a pore diameter D communicate with each other through the pore, and an electrode is provided in each of the two chambers, a step of filling one of the two chambers of the sensor with the measurement target sample;

a step of filling the other of the two chambers of the sensor with a second electrolyte solution to electrically connect the two chambers through the pores;

a step of applying a voltage between two electrodes in each of the two chambers to allow an ionic current to flow between the two electrodes via the pore, and measuring a transient change in the ionic current that occurs each time the antibody-modified particle or the antigen-modified particle passes through the pore as a pulse waveform group consisting of a plurality of pulse waveforms; and

a step of estimating the presence or absence or the concentration of the antigen or the antibody to be detected in the biological sample by analyzing the pulse waveform group;

wherein the blocking size m is equal to or larger than a lower limit determined according to a size of the antigen or the antibody to be detected and is equal to or less than 1/2 of the pore diameter D, and the diameter d of the antibody-modified particle or the antigen-modified particle is 1/5 or more of the pore diameter D and less than the pore diameter D.

The present invention has the effect of enabling highly sensitive, inexpensive, rapid and simple detection and quantification of proteins (or antibodies or antigens).

## BRIEF DESCRIPTION OF THE DRAWINGS

FIG. 1A shows an example of the device structure of a sensor used in the present invention.

FIG. 1B shows an electron microscope image of a pore used to measure a pulse waveform.

FIG. 2 shows an example of a transient change in ion current.

FIG. 3 shows a chart outlining the principle and procedure for detecting or quantifying an antigen in a biological sample based on an embodiment of the present invention.

FIG. 4 shows a chart showing an example of an antibody-modified particle preparation process procedure.

FIG. 5 shows a schematic representation of a chamber filled with a sample to be measured.

FIG. 6 shows a histogram of the number of pulses per peak current of the pulse waveform for measurements at each concentration.

FIG. 7 shows the percentage of the number of pulses of a pulse waveform with a peak current of 0.7 nA or more as an antibody-modified particle aggregate via the target antigen to the total pulses.

FIG. 8 shows the average pulse height when polystyrene standard beads of various particle sizes are passed through the pores of a sensor.

FIG. 9 shows a comparison of pulse waveforms when a biological sample is measured by a conventional method of electrical resistance pore method between the conventional method and the method according to the present invention.

Fig. 10 is a diagram explaining a method for measuring influenza A N protein as a target antigen using culture supernatant from MDCK cells of influenza A (N1H1 A/California/07/2009).

Fig. 11 is a chart explaining the procedure of pre-measurement processing according to the present invention.

FIG. 12 shows the results of measurement using a sensor with pores having a diameter of 305 nm, in which influenza A N protein was extracted as the target antigen from the culture supernatant of MDCK cells, filtered through a 100 kDa filter, and then pre-processed by mixing with 80 nm antibody-modified particles and incubating the mixture.

Fig. 13 shows the distribution of peak currents when a cultured influenza virus sample is measured after pre-measurement processing.

Fig. 14 shows another example of a pulse waveform in a state where an antigen is bound to an antibody-modified particle.

Fig. 15 is a diagram explaining a method for quantifying the blocking size according to the present invention.

## DETAILED DESCRIPTION OF THE INVENTION

The present invention is capable of detecting and quantifying antigens in biological samples, and is also capable of detecting and quantifying antibodies in biological samples. In the former case, antibody-modified particles are used whose surfaces are modified with antibodies that specifically bind to the antigen to be detected or quantified (hereinafter referred to as "target antigen"). In the latter case, antigen-modified particles are used whose surfaces are modified with an antigen that specifically binds to the antibody to be detected or quantified (hereinafter referred to as the "target antibody"). When the antibody-modified particles are used to detect or quantify a target antigen, and when the antigen-modified particles are used to detect or quantify a target antibody, the procedure and principle of the present invention are the same. In order to avoid unnecessarily lengthy description, the following will explain the former example, that is, the detection or quantification of a target antigen using antibody-modified particles.

In another embodiment of the present invention, an antibody in a biological sample can be detected and quantified. That is, this embodiment can be understood by replacing the antigen in the previous description with the antibody and vice versa. Although the explanation will not be repeated below, please note that each description in this specification can be interpreted as described above as appropriate to the extent that there is no contradiction.

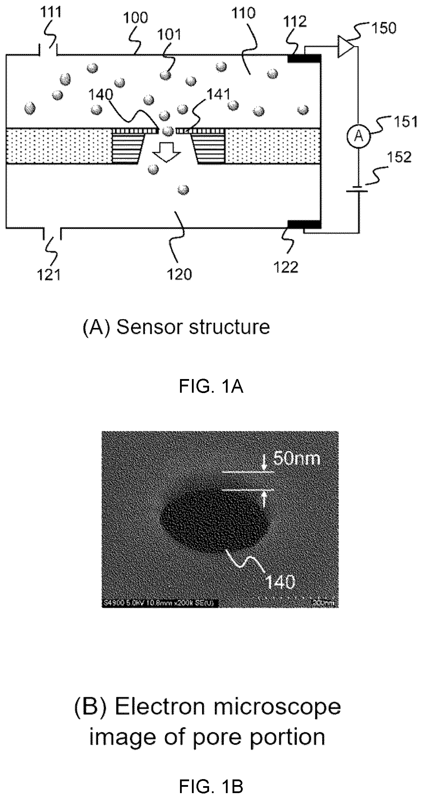

In the present invention, antibody-modified particles, the surface of which is modified with an antibody that specifically binds to the protein to be detected or quantified (hereinafter referred to as the "target antigen" or simply "antigen"), are measured using a sensor such as that shown in FIG. 1 to measure the aggregation state in which the particles bind via the target antigen, the adhesion of the antigen to the antibody-modified particles, or the adhesion state of a secondary antibody to the antibody-modified particles via the antigen, rather than using conventional optical methods.

FIG. 1A shows an example of a cross-section of a sensor used in the present invention. The sensor 100 has a cross-sectional structure in which two chambers 110 and 120 are separated by a partition wall 141 and connected via a pore 140 provided in the partition wall 141. The two chambers are respectively provided with electrodes 112 and 122. A sample containing particles suspended in an electrolyte is introduced into chamber 110 through inlet 111, and the electrolyte is introduced into chamber 120 through inlet 121. A voltage is applied to the two electrodes by voltage source 152. For example, application of a voltage between electrodes 112 and 122 causes an ionic current to flow through the pores. In addition, as used herein, the term "chamber" refers to a portion capable of storing a sample solution (electrolyte solution). In addition, as used herein, an electrode is defined as being "inside the chamber" if it is in a position where it can come into contact with the sample liquid in the chamber (that is, where electricity can be passed therethrough). In addition, as used herein, "filling" the chamber does not necessarily mean filling the entire volume of the chamber, but may leave some void as long as the sensor functions. That is, "filling" may be considered interchangeably herein with "injecting" or "introducing." In addition, in this specification, when the sample itself is liquid, it may be considered as a type of electrolyte. FIG. 1B shows an electron microscope image of a pore 140 used to measure the pulse waveform in the following experiment. The diameter of the pore is selected to be larger than the diameter of the antibody-modified particle to be measured.

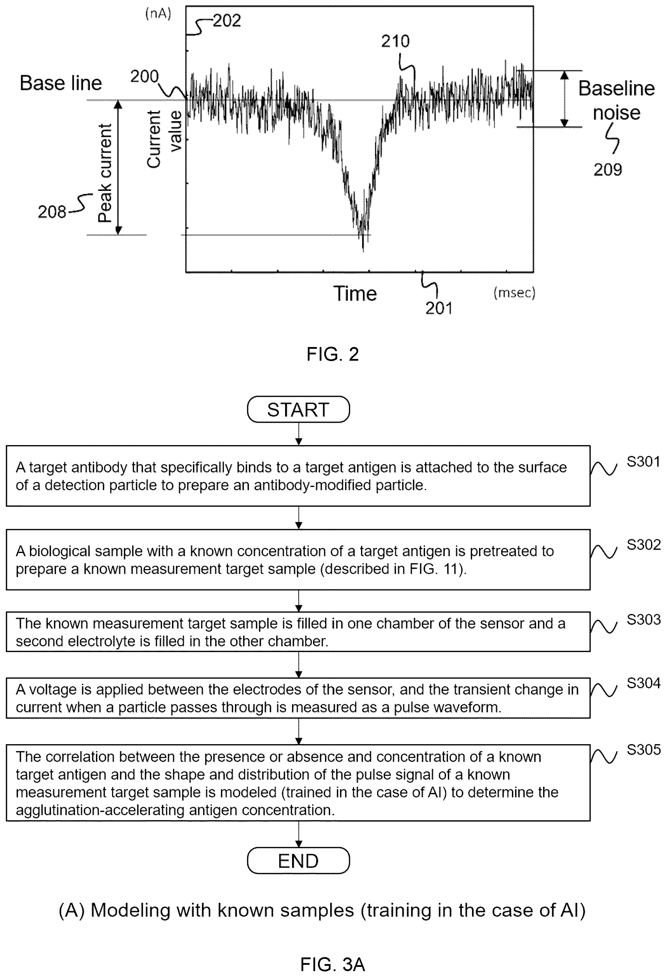

As shown in FIG. 1A, when a particle present in chamber 110 passes through the pore 140, the ion current is temporarily interrupted, and returns to normal after the particle passes through chamber 120. Therefore, every time a particle passes through the pore 140, the ion current flowing between the electrodes in FIG. 1 exhibits a pulse-like transient change as shown in FIG. 2. This is measured by the ammeter 151. In the example shown in FIG 2, the pulse waveform is an example of a current value 202 at each time 201. In another embodiment, the vertical axis 202 may be a voltage value. In the following, an example of an object to be analyzed for a pulse waveform or its distribution is a peak current value 208. A baseline noise 209 is a noise width when there is no pulse.

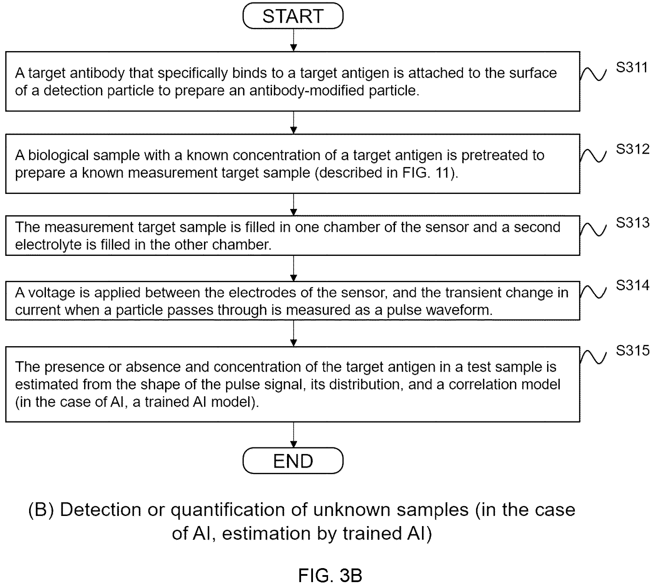

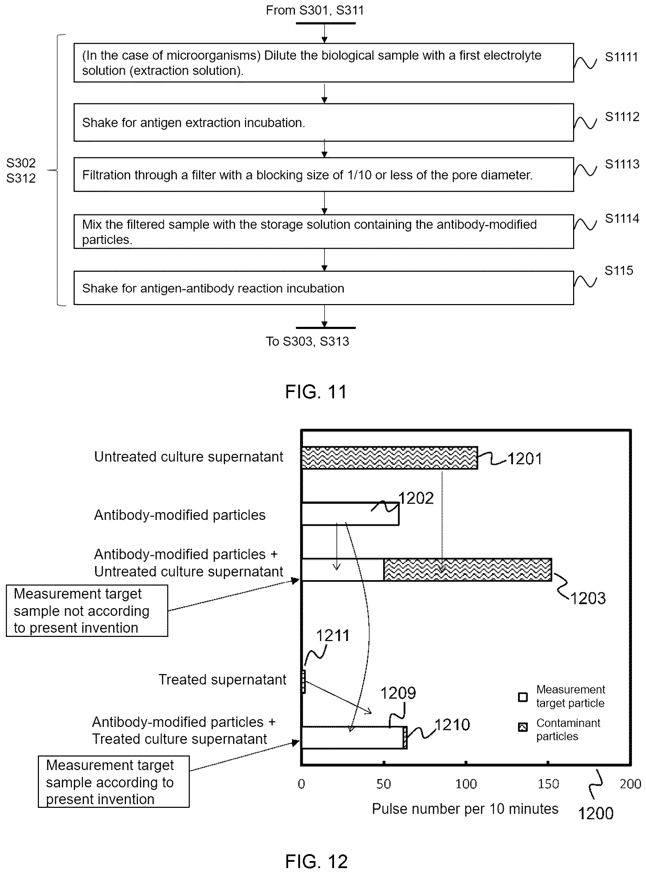

FIG. 3 shows a chart outlining the principles and procedures for detecting or quantifying an antigen in a biological sample according to one embodiment of the present invention. In the present invention, before the unknown biological sample, a known sample in which the presence or absence and concentration of a target antigen are known is measured as shown in FIG. 3A, and the correlation between the presence or absence and concentration of the target antigen and the shape of the pulse waveform or its distribution characteristics obtained by the method of the present invention is modeled. Alternatively, the shape of the pulse waveform or its distribution characteristics can be used as teaching data to train the AI, creating a trained AI. Thereafter, as shown in FIG. 3B, an unknown biological sample in which the presence or absence and concentration of the target antigen are unknown is measured and compared with the model to detect and quantify the target antigen in the biological sample. Alternatively, the pulse waveform shape or its distribution characteristics of an unknown sample can be input into a trained AI to estimate the detection and quantification of the target antigen. In this specification, the procedure for preparing a sample to be injected into a chamber of a sensor as shown in FIG. 1 (hereinafter referred to as a measurement target sample) from a biological sample collected from a living body is referred to as "pre-measurement processing". Since the pre-measurement processing is the essence of the present invention, it will be explained in detail with reference to FIG. 11 after explaining the outline thereof with reference to FIG. 3. In addition, in this specification, a known sample containing a target antigen of a known antigen concentration and antibody-modified particles whose surface is modified with an antibody that specifically binds to the target antigen may be referred to as a "known measurement target sample." Further, a sample containing an unknown sample containing a target antigen with an unknown antigen concentration and antibody-modified particles whose surfaces are modified with antibodies that specifically bind to the target antigen may be referred to as an "unknown measurement target sample." These measurement samples can be mixed and prepared depending on the reaction efficiency of the target antigen and antibody. These measurement samples may be interpreted as including an electrolyte for electrically connecting the chambers. The electrolytes injected into both chambers may have different compositions or may have the same composition. Any electrolyte known in the art may be used depending on the application of the present invention.

In the method shown in FIG. 3A, first, an antibody that specifically binds to a target antigen is modified onto a detection particle to prepare an antibody-modified particle (step S301). Next, a biological sample in which the presence or absence and concentration of a target antigen are known is subjected to a measurement preprocessing step as described with reference to FIG. 11 to prepare a known measurement target sample (step S302). The known measurement target sample contains a first electrolyte solution. Next, one chamber of sensor 100 is filled with a known measurement target sample, and the other chamber is filled with a second electrolyte solution, thereby establishing electrical continuity between electrodes 112 and 122 (step S303). In addition, the first and second electrolytic solutions may have the same composition, or may be different compositions.

Thereafter, a voltage is applied between the electrodes of the sensor, and a pulse waveform due to the passing of the particle is measured (step S304). Then, a model is created of the correlation between the presence or absence and concentration of the known target antigen and the individual shapes and distribution of the pulse waveforms obtained in step S304 above. This modeling may be done analytically or by training an AI model. The AI model is trained using the pulse waveforms themselves, pulse features that represent the characteristics of each pulse waveform, and distribution features that represent the distribution characteristics of the characteristics of a pulse waveform group obtained in a single measurement as teacher data, and also using the presence or absence and concentration of known target antigens as teacher labels. When using an AI model, any algorithm may be used, such as a support vector machine, a linear discriminant transform, a k-nearest neighbor method, a decision tree, or an ensemble learning of these, or various deep learning and recursive algorithms. Alternatively, it may be an algorithm in which the model itself changes dynamically based on the learning results, such as reinforcement learning. When examining whether or not a target antigen is contained in a biological sample using the method shown in FIG. 3B, a classification algorithm that outputs the presence or absence may be used. In addition, when quantifying the target antigen using the method shown in FIG. 3B, a regression algorithm or the like that outputs a continuous amount may be used.

After the above modeling (or creation of a trained AI), the measurement results of a biological sample in which the presence or absence and concentration of a target antigen are unknown are compared with the model (or trained AI) to estimate the presence or absence and concentration of the target antigen, as illustrated in FIG. 3B. The processes in steps S311 and S312 are the same as those in FIG. 3A, and therefore the description thereof will be omitted. It is necessary to use the same antibody-modified particles in steps S301 and S311.

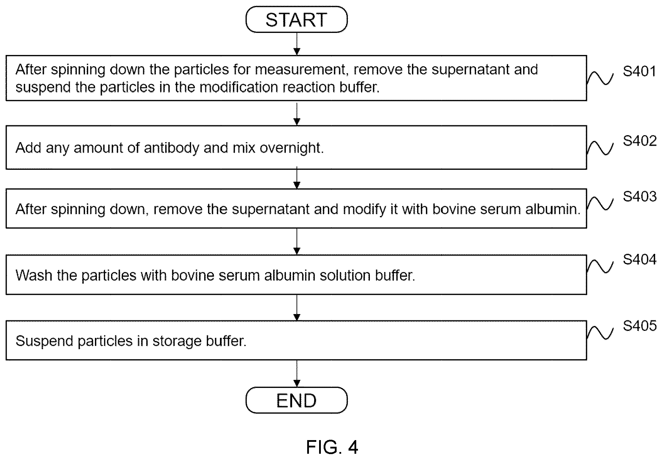

An example of the antibody-modified particle production process in steps S301 and S311 is shown in FIG. 4. In FIG.4, antibody-modified particles were created using physical adsorption to polystyrene particles utilizing hydrophobic interactions, but this is merely one example of a protocol in the present invention, and antibody-modified particles may be created using any principle or method, such as carbodiimide bonding. With this treatment, the measurement particles that serve as the substrate for modification can be made of any material, including high molecular weight polymers and metals. In addition, the type of antibody to be modified can be any type, whether monoclonal, polyclonal, or recombinant, as long as it specifically binds to the target antigen.

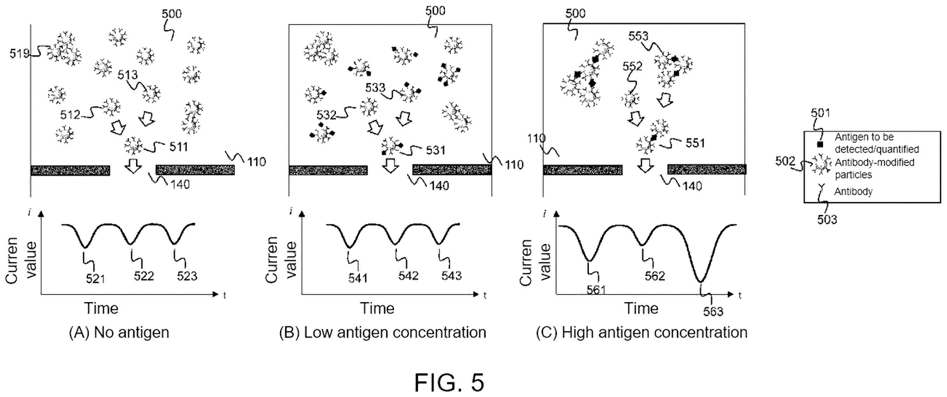

Next, the basic principle of detection or quantification of a target antigen in the present invention will be described. FIG. 5A to FIG. 5C are schematic diagrams showing a state in which the chamber 110 is filled with a sample to be measured. When the biological sample does not contain the target antigen, as shown in FIG. 5A, the target antigen bound to the target antibody is not present in the measurement sample. When a voltage is applied between the electrodes 112 and 122 in this state, most of the antibody-modified particles in the measurement sample pass through the pore 140 alone, as shown in FIG. 5A. In the example of FIG. 5A, the pulse waveforms when antibody-modified particles 511, 512 and 513 pass through the pore 140 are 521, 522 and 523, respectively. If the biological sample does not contain the target antigen, the antibody-modified particles will not bind via the target antigen. For this reason, most of the antibody-modified particles pass through the pores alone, but some of them may bind nonspecifically without the target antigen, such as particle 519, and the antibody-modified particles may be observed passing through the pores in a bound state.

When a target antigen is present in the biological sample, the target antigen is loaded into chamber 110 in a state in which it is bound to antibody-modified particles, for example, as shown in FIG. 5B and FIG. 5C. FIG. 5B shows the case where the antigen is dilute, and although the target antigen is bound to the antibody-modified particles, there is almost no aggregation between the antibody-modified particles via the target antigen.

As shown in FIG. 5C, when the concentration of the target antigen is high, the antibody-modified particles form aggregates via the target antigen. Conventional techniques such as turbidimetry, absorbance method, and immunochromatography utilize this agglutination phenomenon. In the present invention, the pulse waveform generated when an aggregate passes through the pore has a greater peak current value than the pulse waveform generated when an antibody-modified particle passes through the pore alone. This is because when a large particle passes through the pore, the ionic current through the pore is impeded to a greater extent than when a small particle passes through the pore. For example, in FIG. 5C, the pulse waveforms of agglomerate 551, particle 552 and agglomerate 553 passing through aperture 140 are 561, 562 and 563, respectively. This is the reason why the peak current value of the pulse waveform is greater for agglomerate 551 than for single particle 552, and is further greater for agglomerate 553. On the other hand, in the state of Figure 5B, since no aggregates are formed, the peak current values of pulse waveforms 541 to 543 are almost the same as in the state of FIG. 5A. However, since the charge changes due to the binding of the antigen to the antibody-modified particle, a change occurs in the pulse waveform. As a result, in the present invention, it is possible to detect and quantify the target antigen even in the state of FIG. 5B.

In the present invention, the shape of the pulse waveform is statistically analyzed to estimate the degree of adhesion of the target antigen to the antibody-modified beads shown in FIG. 5B and the degree of aggregation between the antibody-modified particles via the target antigen shown in FIG. 5C. This allows detection and quantification of the target antigen in the sample to be measured.

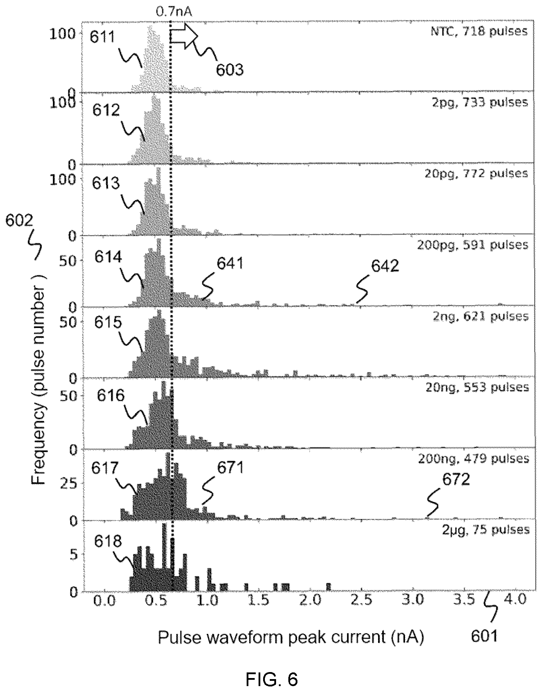

To demonstrate the effectiveness of the present invention, the present inventor first prepared a 10-fold dilution series of a recombinant N protein sample of influenza A virus from 2 μg/mL to 2 pg/mL, rather than a biological sample, and then measured the pulse waveform with the sensor shown in FIG. 1 as an example, using the N protein as the target antigen. The pore diameter was about 305 nm, and the diameter of the antibody-modified particles was about 80 nm. FIG. 6 shows a histogram of the number of pulses 602 for each peak current 601 of the pulse waveform for measurements at each concentration. The peak current of the pulse waveform is 208 in FIG. 2. NTC is a measurement target sample that does not contain the target antigen. Therefore, the pulse measurement result of the histogram 611 is considered to be approximately the distribution of pulse waveforms when the antibody-modified particle alone in the state shown in FIG. 5A passes through the pore 140. FIG. 6 shows histograms 612 to 618 of peak currents of pulse waveforms for samples with target antigen concentrations of 2 pg/mL, 20 pg/mL, 200 pg/mL, 2 ng/mL, 20 ng/mL, 200 ng/mL and 2 μg/mL. The shapes of the histograms at 2 pg/mL and 20 pg/mL are almost unchanged from that of NTC, and these are considered to be in the state shown in FIG. 5B. On the other hand, when the target antigen concentration was 200 pg/mL, pulse waveforms with large peak currents, such as pulses 641 and 642, which are likely due to aggregates, were observed, and these increased as the target antigen concentration increased.

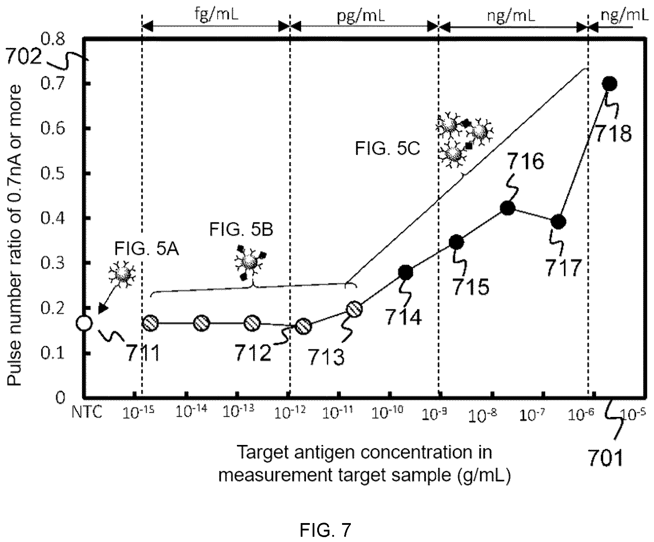

FIG. 7 shows the pulse waveform with a peak current of 0.7 nA or more 603 as the antibody-modified particle aggregate via the target antigen, and the ratio of the number of pulses to all pulses. The horizontal axis 701 is the target antigen concentration, and the vertical axis 702 is the ratio of pulses of 0.7 nA or more to all pulses. Reference numerals 712 to 718 in FIG. 7 correspond to 612 to 618 in FIG. 6. In one example of this measurement result, aggregation of the antibody-modified particles progresses from approximately 20 pg/mL 713 to 200 pg/mL 714, and therefore it can be seen that in the antigen concentration range above this, the state is as shown in FIG. 5C. In the range of 2 pg/mL to 20 pg/mL or less, the antigen is gradually attached to the antibody-modified particles, which is considered to be in the state shown in FIG. 5B. In the present invention, it is possible to detect or quantify the target antigen by estimating the degree of antigen attachment to the antibody-modified particle in the state of FIG. 5B and the aggregation state of the antibody-modified particle in the state of FIG. 5C, from the characteristics of the pulse waveform and its distribution characteristics.

As described above, basic principle of the present invention has been explained above with reference to FIG. 5. In addition, the principle has been demonstrated with a dilution series of recombinant N protein of influenza A virus with reference to FIGS. 6 and 7. However, the experiments in FIGS. 6 and 7 use pure line samples that contain almost no contaminants. For this reason, most of the obtained pulse waveforms were of free or aggregated antibody-modified particles, and it was easy to model the correlation between the presence or absence and concentration of the target antigen and the characteristics of the pulse waveform and its distribution characteristics (step S305).

However, biological samples intended for clinical application contain many contaminants. For example, blood and urine, which are often used as clinical specimens, contain many cells such as blood cells and their fragments. In addition, saliva and nasopharyngeal fluid, which are used as specimens in infectious disease tests, contain a small number of blood cells but a large number of exosomes of about several hundred nm in size. Since it is not practical to sort these particles on the order of μm to nm, in order to realize detection and quantification of target antigens using the basic principle shown in FIG. 5, a technology is required that can efficiently eliminate pulse waveforms originating from contaminants other than the target antibody-modified particles.

The present inventors have discovered that the measurement pre-processing technique of the present invention, which utilizes the filtering and pore characteristics described below, can largely eliminate pulse waveforms other than those resulting from the passage of the antibody-modified particles to be measured through the pore, thereby making it possible to detect or quantitate the target antigen with high accuracy, and have completed the present invention. That is, according to the present invention, assuming the pore diameter of the sensor pore used when measuring the transient change in current when particles pass through is D, the blocking size of the filter used in the pre-measurement processing described in detail below is m, and the particle size of the antibody-modified particles to be prepared is d, then both of the following formulas (A) and (B) are satisfied.

[Equation 1]

$$0.2D \leq d < D \qquad (A)$$

[Equation 2]

$$M_{min} \leq m \leq 0.5D \qquad (B)$$

In which, $M_{min}$ is a lower limit determined according to the size of the antigen to be measured. In one embodiment, $M_{min}$ can be set to, for example, 2 nm, which is the size of a typical protein. In other words, if the antigen to be measured is blocked by the filter and more than 30% is lost, it will inhibit the subsequent antigen-antibody reaction (that is, the sensitivity of the measurement will decrease), so it is understandable from the standpoint of technical common sense that it is desirable not to make it any smaller. In other embodiments, $M_{min}$ may be scaled to the size of another antigen.

The principles of the present invention and experimental verification results will be described in more detail below.

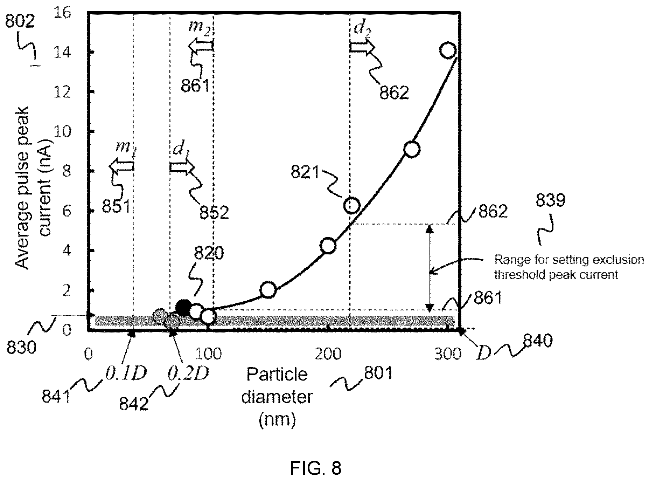

The characteristics of a sensor such as that shown in FIG. 1, which is the basis of the principle of the present invention, will be described with reference to FIG. 8. FIG. 8 shows the average pulse height 802 when standard polystyrene beads of various particle sizes 801, ranging from 60 nm to 300 nm, are passed through a pore with a diameter D of about 305 nm used in measuring the data illustrated in FIG. 6 and FIG. 7. The smaller the particle is, the smaller the average pulse peak current is. This is because the smaller the particle diameter is, the smaller the proportion of passing particles in the pores is, and as a result, the smaller the blockage of the ionic current flowing through the pores is, and the smaller the change in resistance value due to the passage of particles is. In the example of FIG. 8, when the pulse height becomes equal to or less than about 30 nA 830, the pulse becomes buried in baseline noise and cannot be observed. The baseline noise referred to here is the usual noise other than the pulse waveform, such as the example shown as 209 in FIG. 2.

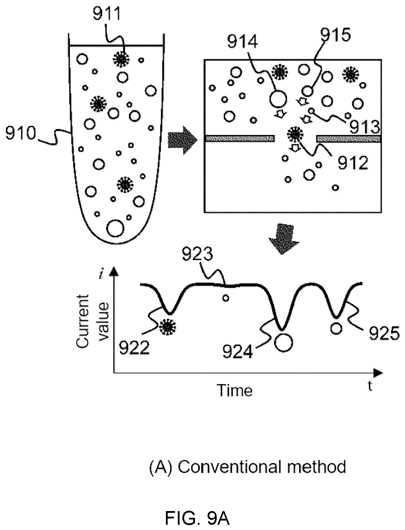

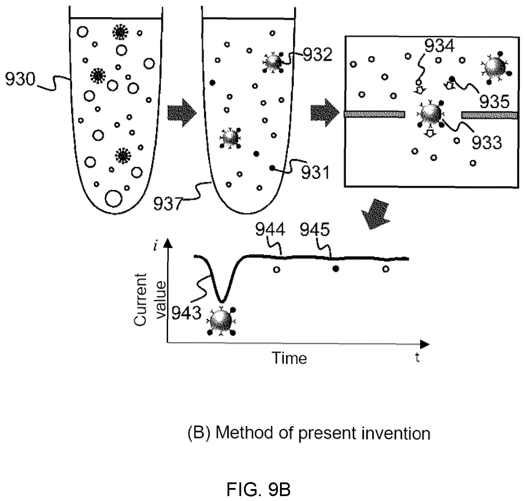

Next, the principle of the contaminant particle removal according to the present invention will be described with reference to FIG. 9. FIG. 9A shows a pulse waveform obtained when a biological sample is measured by the conventional electrical resistance pore method. When the objective is to measure virus particles 911 in a biological sample 910, many pulses of contaminants other than virus particles contained in the biological sample 910 are observed. For example, in FIG. 9A, of particles 912 to 915 that pass through the pore, only particle 912 is a virus particle. However, in the measurement, pulse waveforms such as 922 to 925 are measured, and it is impossible to distinguish which pulses are caused by the virus.

FIG. 9B is a conceptual diagram of the method according to the present invention. In the present invention, in a sample 930 containing many contaminants, first, a protein specific to the virus to be measured is extracted as a target antigen 931 from the virus. Thereafter, the virus and other contaminants of a similar size to the virus are removed using a filter, and then the sample 937 mixed with the antibody-modified particles 932 is measured. The sample 937 contains not only the target antigen, but also many contaminant particles of a similar size. However, as can be seen from FIG. 8, for example, particles smaller than 1/10 of the pore diameter D840 are not observed as pulses. Therefore, particles 933 to 935 passing through the pores become pulses like 943 to 945, respectively, and only the antibody-modified particles to be measured are measured as pulses.

(Embodiment 1)

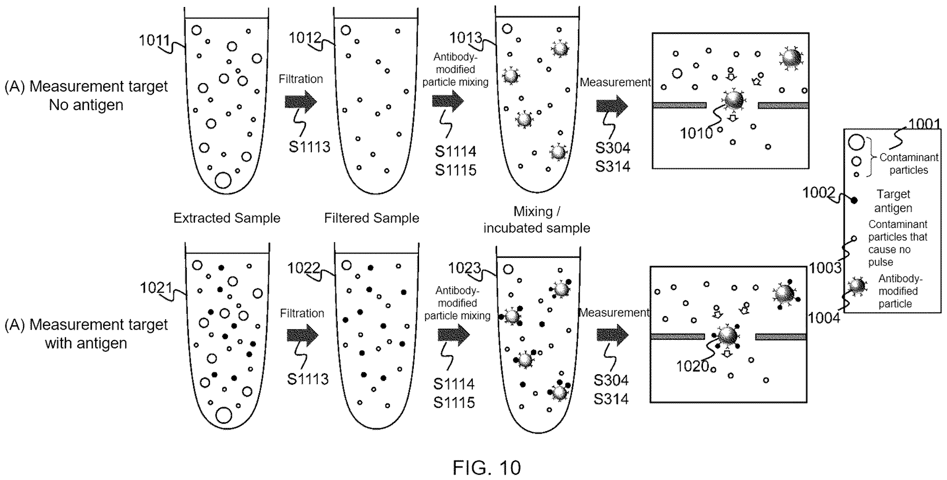

One embodiment of the present invention will be described with reference to FIG. 10 and FIG. 11, taking as an example a method for measuring influenza A N protein as a target antigen by the method according to the present invention using a culture supernatant of influenza A (N1H1 A/California/07/2009) from MDCK cells. When measuring microorganisms as shown in FIG. 9, first, a process for extracting proteins specific to the microorganisms to be detected or quantified is performed (steps S1111 and S1112). In this example, the first electrolyte solution was a solution in which PEG (Polyethylene glycol) and a surfactant were diluted with PBS (Phosphate-buffered saline). In this example, steps S1111 and S1112 are performed to extract the N protein, which is the target antibody inside the influenza virus, but these processes are appropriately selected depending on the type of biological sample and target antigen. In addition, when the target antigen is already in a state capable of reacting with the antibody-modified particles within the biological sample, such an extraction process is not necessary, and the sample may simply be diluted with an electrolyte solution such as PBS.

After this treatment, both extracted sample 1011 in which the target antigen is not present and extracted sample 1021 in which the target antigen is present contain a mixture of many contaminant particles and the target antigen. If this is measured as is by a sensor, a noise pulse due to the contaminant particles will be measured as shown in FIG. 9A. Therefore, filtration is performed using a filter with a blocking size of 1/10 or less of the pore diameter (step S1113). In this example, centrifugal filtration was performed at 10,000 G for 3 minutes using a 100 kDa membrane filter. Here, the G and time of centrifugal filtration are merely examples, and optimal values may be selected depending on the biological sample and the extraction process. As a result, as in filtered samples 1012 and 1022, both contaminant particles and microorganisms to be detected/quantified are removed, and particles larger than the filter's blocking size are generally removed. In this specification, the upper limit of particle size at which 30% or more of particles in a sample are removed is defined as the "blocking size" of a filter. For example, a filter that blocks 30% or more of particles larger than 100 nm, but does not block 30% of particles smaller than 100 nm (that is, 30% or more of the particles will pass through) is said to have a blocking size of 100 nm. The filter used in step S1013 may be of any type, such as one that controls the particle size passing through the fibers or one that utilizes the diameter of porous holes. In addition, in this specification, the "blocking size" is defined as blocking "30% or more" because, in general, complete blocking is not realistic with filters made of fibers, and the inventors have discovered that a filter's performance can be practically expressed by blocking 30% or more. The method for quantifying the blocking size will be discussed later.

In addition, in the example of FIG. 11, in step S1111, the first electrolytic solution is diluted with an electrolytic solution having a function of extracting N protein, which is a target antigen from microorganisms. The sample to be measured must contain the first electrolyte solution, but the process of diluting with the electrolyte solution is not limited to step S1111, and may be performed anywhere in S1111 to S1115, and in any order.

In the present invention, in a sensor such as that shown in FIG. 1, both chambers 110 and 120 must be filled with an electrolyte in order to allow an ionic current to flow through the pores. In this example, the sample filling chamber 110 was mixed with a first electrolyte solution in step S302 (S1111), and chamber 120 was filled with a second electrolyte solution. In this method, the first electrolytic solution needs to have components necessary for efficiently producing and maintaining the state of FIG. 5B or FIG. 5C, and the second electrolytic solution only needs to realize stable conduction. For example, the first electrolyte may contain a component such as PEG that promotes antigen-antibody reactions, while the second electrolyte may not contain a component that affects the function of the antigen or antibody, such as a high concentration of a surfactant. Since the chamber 120 only serves to conduct ion current, it does not need any functions as the chamber 110 and is not limited thereto. For example, the second electrolyte may contain a surfactant at any concentration for stable conduction. That is, in this embodiment, the first electrolyte and the second electrolyte may have different compositions. In another embodiment, the first electrolyte and the second electrolyte may have the same composition.

In the example shown here, a 305 nm diameter pore was used for the measurement. The size of the target antigen is approximately 2.7 nm, assuming a molecular weight of 60 kDa. Therefore, the target antigen will pass through a filter with a blocking size of 1/10 or less of the pore diameter (that is, a filter with a blocking size of 30.5 nm or less), but contaminant particles with a particle size of 61 nm or more, which is 1/5 or more of the pore diameter, will be largely removed (that is, more than 30% of these contaminant particles will be blocked and removed by the filter). Next, antibody-modified particles having a particle size equal to or larger than 1/5 of the pore diameter of the pore 140 created in S301 or S311 are mixed with the filtered sample 1012 or 1022 (step S1114). Further, incubation for antigen-antibody reaction is performed (step S1115). This completes measurement target sample 1013 or 1023, and the process proceeds to step S303 or S313 in FIG. 3.

In the first embodiment illustrated here, if the pore diameter of the sensor pore 140 used in step S304 or S314 is D, the blocking size of the filter used in step S1113 is $m_1$, and the particle size of the antibody-modified particle created in step S1114 is $d_1$, then measurement is performed under conditions that satisfy both the following formulas (1) and (2). As an example, $M_{min}$ may be 2 nm.

[Equation 3]

$$0.2D \le d_1 < D \qquad (1)$$

[Equation 4]

$$M_{min} \le m_1 \le 0.1D \qquad (2)$$

In this embodiment, the reason why detection or quantification of a target antigen in a biological sample containing many contaminants can be achieved with high accuracy by satisfying the conditions of formulas (1) and (2) will be explained. By the process of step S1113 in FIG. 11, particles having a particle size m1 or more shown in (2) are mostly removed from the sample. On the other hand, since there is no method for selectively removing the contaminant particles close in size to the target antigen from a biological sample, the filtered samples 1012 and 1022 after processing in step S1113 still contain a large amount of contaminant particles, some of which contain the target antigen, even after filtration. However, if the filtration satisfies formula (2), no pulse will be measured even if the contaminating particles and the target antigen of the sample are measured. Furthermore, in the samples 1013 and 1023 in which antibody-modified particles satisfying formula (1) are mixed and incubated, the pulse of only the antibody-modified particles or their aggregates to be measured can be measured.

In this way, the present invention can measure the presence or absence of target antigen binding to antibody-modified particles 1010 and 1020 in FIG. 10, and the difference in the agglutination state of antibody-modified particles due to the target antigen, without noise pulses. This makes it possible to detect and quantify target antigens even in biological samples containing large amounts of contaminants with the same performance as in pure samples containing only the target antigen. In addition, when the filter used in filtration S1113 is fibrous, it is inevitable that particles larger than the blocking size will pass through the filter and remain in the filtered sample. For this reason, in this embodiment, as shown in FIG. 8, the blocking size 841 in formula (2) is set to be smaller than the lower particle size limit 842 of the antibody-modified particles in formula (1). In formulas (1) and (2), it is preferable to set $m_1$ to be smaller than the antibody-modified particle diameter $d_1$, assuming the use of a membrane filter that allows some particles of a blocking size or larger to pass through. In the present invention, the blocking size and the antibody-modified particle diameter are selected so that $m_1$ is smaller and $d_1$ is larger than the particle diameter limit at which a pulse can be detected in measurement with a sensor such as that shown in FIG. 1.

Next, the effect of the method of the present invention on the measurement results of the influenza A culture supernatant will be examined using FIG. 12. FIG. 12 shows the results of pre-measurement processing in which the N protein of influenza A was extracted from the culture supernatant of MDCK cells using influenza A as the target antigen, filtered through a filter with a blocking size of 30 nm, and then mixed with 80 nm antibody-modified particles and incubated, followed by measurement using a sensor with pores with a diameter of 305 nm. The blocking size of the above filter satisfies formula (2) for the pore diameter D of 305 nm. Moreover, the antibody-modified particles with a diameter of 80 nm used in this measurement satisfy (1). For these reasons, the biological sample that has been subjected to this pre-measurement treatment is in the state of 1013 or 1023 in FIG. 10.

In FIG. 12, the horizontal axis 1200 represents the number of pulses per 10 minutes. The number of pulses 1201 represents the result of measuring the pulse waveform of an extracted sample 1021 of a culture supernatant containing a target antigen. Since the target antigen, influenza N protein, is not observed as a pulse with the 305 nm pore, this pulse is entirely due to contaminating particles. In addition, the pulse number 1202 is the result of performing pulse measurement on the antibody-modified particle storage solution mixed in step S1114. The pulse measurement result of sample 1023 obtained by mixing and incubating these is pulse number 1203. The number of pulses is roughly the sum of 1201 and 1202, but many of the pulses are due to contaminating particles, and it is difficult to model small differences such as antigen attachment to the antibody-modified particle surface.

In contrast, the pulse number 1211 is the pulse measurement result of the filtered sample 1022 in step S1113, and almost no pulses are measured. Therefore, the pulse measurement result of sample 1023, which was mixed with antibody-modified particles and incubated, is as shown by 1210, and almost all of the pulses are due to the antibody-modified particles. This shows that according to the present invention, even in biological samples containing many contaminants such as clinical specimens, target antigens can be detected and quantified in the same manner as in the pure systems shown in FIG. 6 and FIG. 7.

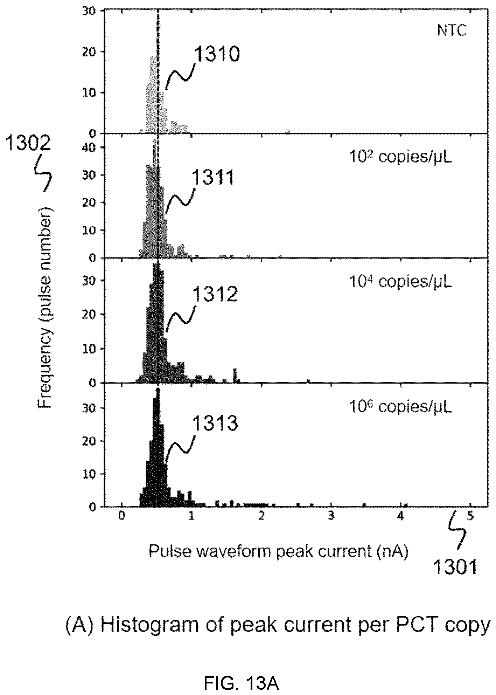

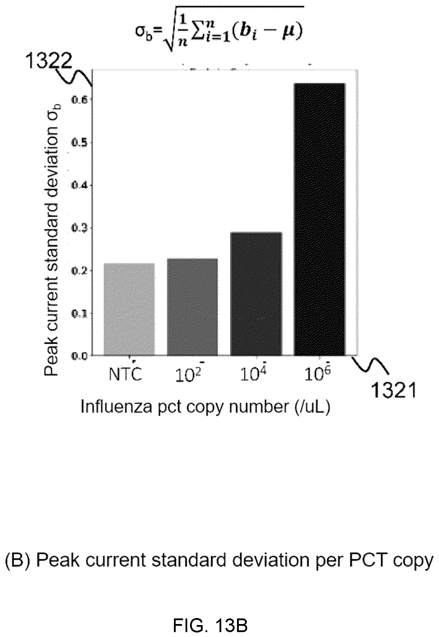

FIG. 13 shows the distribution of peak currents when a cultured influenza virus sample was subjected to the pre-measurement treatment shown in FIG. 11 under the conditions of formulas (1) and (2) and then measured by the method of the present invention. NTC indicates culture supernatant not containing influenza virus, and the following distributions 1311 to 1313 indicate the results when the PCR copy numbers were $10^2$, $10^4$ and $10^6$ copies/$\mu$L, respectively. By the pre-measurement process shown in FIG. 11, most of the pulses in this histogram are due to antibody-modified particles, similar to the case of FIG. 6 measured in a pure system. The results, similar to those shown in FIG. 6, show that, as compared to NTC1310 not containing influenza virus, pulses of large peak current appear with each increase in the virus concentration in the sample. FIG. 13B shows the standard deviation of this distribution. By modeling the correlation between such a pulse waveform distribution and the N protein of influenza A, which is the target antigen, it is possible to estimate the influenza virus concentration in the culture supernatant, which is the biological sample.

As described above, specific proteins were extracted from microorganisms in biological particles containing a large amount of contaminating particles, and were then used as antigens to perform quantification using the method of the present invention. The method of the present invention is effective not only in microorganisms, but also as long as antigenic proteins are contained in biological samples.

(Embodiment 2)

In the above-mentioned embodiment 1, the pore diameter D, the particle diameter $d_1$, and the blocking size m1 were selected so that almost no pulse was measured from the sample after filtration. An example is the number of pulses 1211 in FIG. 12. Meanwhile, in another embodiment of the present invention, the pore size, particle size, and blocking size may be selected so that pulses are measured from the filtered sample, and pulse waveforms with peak currents smaller than an exclusion threshold may be excluded from the analysis.

In one example of this embodiment, the blocking size $m_2$ (861) and the antibody-modified particle diameter $d_2$ (862) in FIG. 8 are selected and measured so as to satisfy the following formulas (3) and (4).

[Equation 5]

$$d_2 < D \qquad (3)$$

[Equation 6]

$$M_{min} \leq m_2 \leq 0.5d_2 \qquad (4)$$

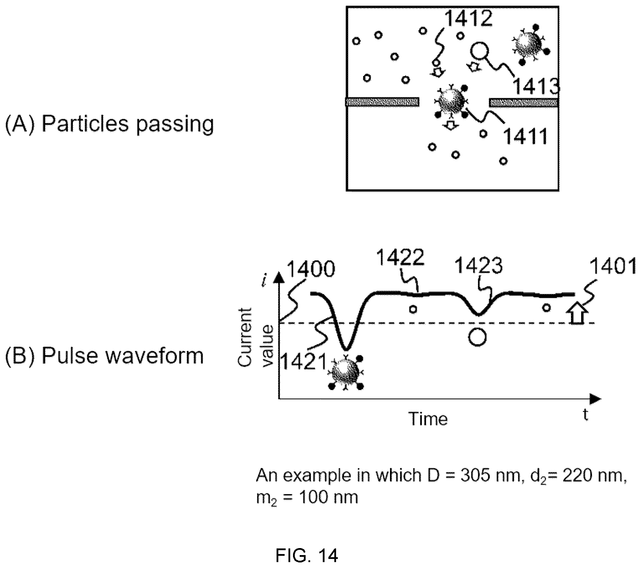

Here, as an example, if D = 305 nm, $d_2$ = 220 nm, $m_2$ = 100 nm, and $M_{min}$ = 2 nm as shown in FIG. 14, the measurement data will show a mixture of antibody-modified particles and contaminant particles, as shown in FIG. 14. In the example of FIG. 14, as particles 1411 to 1413 pass through the pore, pulse waveforms 1421 to 1423 are measured, respectively. In this example, even if the particle 1413 is smaller than the stopping size $m_2$, the pulse is measured as waveform 1323. In this embodiment, an exclusion threshold 1400 is set, and pulse waveforms such as pulse waveform 1423 whose peak current falls below the exclusion threshold 1400 are excluded from the modeling or estimation in step S305 or S315. The exclusion threshold may be, for example, within range 839 in FIG. 8, but may also be selected recursively, for example to best perform the evaluation function of the correlation modeling of step S305. Also, for example, when training an AI model in step S305, the exclusion threshold 1400 may be determined so that the output of the trained AI model best reproduces the presence or absence and antigen concentration of a known sample.

In this embodiment, the measurement result of the filtered sample will be a mixture of pulses originating from the antibody-modified particles and the contaminant particles. However, as long as formulas (3) and (4) are satisfied, the peak current of the pulse waveform derived from contaminants is significantly smaller than that of the antibody-modified particles. Therefore, by excluding these, only the antibody-modified particles can be analyzed. For example, in cases where a filter with a small blocking size is expensive, where an expensive centrifugal device is required, or where there are problems such as a long filtration time, it may be difficult to comply with formulas (1) and (2). In such cases, the method according to the second embodiment is effective.

In the above description of the first and second embodiments, in FIG. 9, FIG.10 and FIG. 14, detection of a state in which an antigen is bound to an antibody-modified particle, that is, the state shown in FIG. 5B, is taken as an example. The present invention can also be used to detect the state shown in FIG. 5C in which antibody-modified particles are aggregated together by the target antigen.

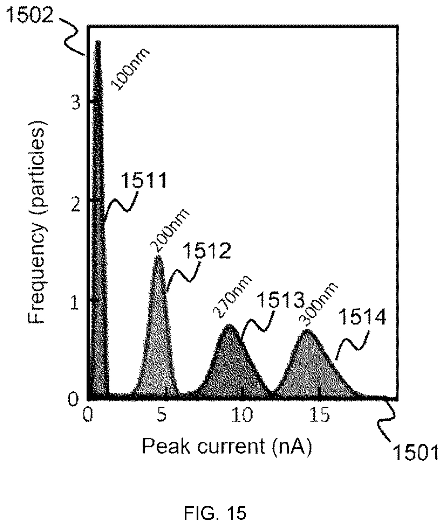

The blocking size of the above-mentioned filter can be evaluated by using standard beads with known particle sizes and measuring the pulse waveform using the method described above. FIG. 15 shows a histogram of the peak current distribution obtained by measuring a pulse waveform using a sensor such as the sensor 100 described above using standard beads conforming to the NIST (National Institute of Standards and Technology) and having a known particle size. Distributions 1511 to 1514 are the results of measuring standard beads of 100 nm, 200 nm, 270 nm, and 300 nm using a sensor having a 305 nm pore diameter as exemplified in FIG. 1, and the distributions are clearly different for each particle diameter. By measuring samples of standard beads of each particle size filtered through the filter being evaluated and comparing the number of pulses with the measurement results for samples not filtered through the filter, the blocking rate for each particle size can be estimated. The particle size at which the blocking rate is 30% or more is defined as the blocking size.

Furthermore, the present invention has been described above using an example in which antibody-modified particles that specifically bind to a target antigen are used as the measurement target, and the target antigen is detected or quantified in biological particles containing a large amount of contaminants. Without being limited thereto, the present invention can also measure antigen-modified particles, the surface of which is modified with an antigen that binds to an antibody to be detected or quantified, to detect or quantify a target antibody in a biological particle containing a large amount of contaminants. In this case, the antibody and antigen in the above explanation can be read inversely.

Generally, in conventional techniques for detecting or quantifying an antigen or antibody, it is necessary to capture an antibody or antigen by utilizing the specificity of an antigen-antibody reaction, and then remove as much of the other substances as possible by washing or the like, which poses the problem that removing unnecessary substances requires a lot of effort. What makes this invention unique is that it does not simply remove contaminants from a target sample, but rather it successfully reduces the effort and cost required to remove contaminants by providing conditions under which even if a target sample contains a large amount of contaminants, these do not affect the analytical results.

Furthermore, the above-described method according to the present invention can be implemented by a computer device or terminal having a processor (which may be a single processor or a multi-core processor, and may also include a microprocessor). Such a computer device may be a device integrated with a sensor such as sensor 100 in FIG. 1. Alternatively, the computer device may be a computer device operatively connected to such a sensor via a network (whether wired or wireless, which may be, for example, the Internet, a private closed network, a LAN, or the like).

The computer device that can be used in relation to the present invention may take any form, such as a workstation, a tablet, a smartphone, and the like.

In one embodiment of the present invention, a computer-readable program and a medium for storing the program can be provided, and the program can be executed by a processor to perform any of the steps included in the above-described method.

In some embodiments of the present invention, a device may also be provided for carrying out the above-described methods and for inferring the presence or concentration of an antigen or antibody. That is, the device may include a filter having a blocking size m as described above, a sensor having a structure in which two chambers separated by a partition having a pore with a pore diameter D communicate with each other through the pore and each of the two chambers has an electrode, and an interface connecting to a computer via a network. The interface of the device may be configured to transmit data obtained by measuring the transient changes in ionic current that occur each time an antibody-modified particle passes through the pore as a pulse waveform group consisting of a plurality of pulse waveforms to a computer. The computer may be in any form having a processor, and may be integrated with the device, or may be an external computer connected to the device via some network.

**Claims**

1. A method for estimating presence or absence or a concentration of an antigen or an antibody to be detected in a biological sample collected from a living body, comprising the steps of:

   filtering the biological sample through a filter having a blocking size m to prepare a filtered sample;
   preparing a measurement target sample by mixing:

   antibody-modified particles having a particle diameter d and an antibody that binds to the antigen attached to its surface, or antigen-modified particles having a particle diameter d and an antigen that binds to the antibody attached to its surface,
   the filtered sample, and
   a first electrolyte solution;

   to a sensor having a structure in which two chambers separated by a partition wall having a pore with a pore diameter D communicate with each other through the pore, and an electrode is provided in each of the two chambers, filling one of the two chambers of the sensor with the measurement target sample;
   filling the other of the two chambers of the sensor with a second electrolyte solution to electrically connect the two chambers through the pores;
   applying a voltage between two electrodes in each of the two chambers to allow an ionic current to flow between the two electrodes via the pore, and measuring a transient change in the ionic current that occurs each time the antibody-modified particle or the antigen-modified particle passes through the pore as a pulse waveform group consisting of a plurality of pulse waveforms; and
   estimating the presence or absence or the concentration of the antigen or the antibody to be detected in the biological sample by analyzing the pulse waveform group;
   wherein the blocking size m is equal to or more than a lower limit determined according to a size of the antigen or the antibody to be detected and is equal to or less than 1/2 of the pore diameter D, and
   wherein the diameter d of the antibody-modified particle or the antigen-modified particle is 1/5 or more of the pore diameter D and less than the pore diameter D.

2. The method according to claim 1, wherein the blocking size m is 2 nm or more and 1/10 or less of the pore diameter D, and a diameter $d_1$ of the antibody-modified particle or the antigen-modified particle is 1/5 or more of the pore diameter D and less than the pore diameter D.

3. The method according to claim 1 or 2, wherein an aggregation state of the antibody-modified particles or the antigen-modified particles in the biological sample is measured by measuring the transient change in the ionic current as a group of pulse waveforms consisting of a plurality of pulse waveforms.

4. The method according to claim 1, further comprising:

   extracting a selected pulse waveform having a peak current equal to or more than an exclusion threshold from pulse waveform group; and
   detecting the presence or absence of the antigen or the antibody to be detected in the biological sample or calculating an concentration estimate value thereof by analyzing the selected pulse waveform group;
   wherein the diameter d of the antibody-modified particle or the antigen-modified particle is less than the pore diameter D, and the blocking size m is 1/2 or less of the diameter d.

5. The method according to claim 4, further comprising: recursively determining the exclusion threshold value so that the concentration estimate value is close to the true value of the antigen or the antibody to be detected contained in the biological sample.

6. The method according to any one of claims 1, 2, and 4, wherein the first electrolyte solution and the second electrolyte solution have different compositions.

7. The method according to any one of claims 1, 2, and 4, wherein the first electrolyte solution and the second electrolyte solution have the same composition.

8. A device for estimating presence or absence or a concentration of an antigen or an antibody to be detected in a biological sample collected from a living body, comprising:

a filter having a blocking size m;
a sensor having a structure in which two chambers separated by a partition wall having a pore with a pore diameter D communicate with each other through the pore, and an electrode is provided in each of the two chambers;
an interface for connecting to a computer via a network;

wherein the device is configured to:

prepare a measurement target sample by mixing:

antibody-modified particles having a particle diameter d and an antibody that binds to the antigen attached to its surface, or antigen-modified particles having a particle diameter d and an antigen that binds to the antibody attached to its surface,
the filtered sample, and
a first electrolyte solution;

fill one of the two chambers of the sensor with the measurement target sample;
fill the other of the two chambers of the sensor with a second electrolyte solution to electrically connect the two chambers through the pore; and
apply a voltage between two electrodes in each of the two chambers to allow an ionic current to flow between the two electrodes via the pore, and measure a transient change in the ionic current that occurs each time the antibody-modified particle or the antigen-modified particle passes through the pore as a pulse waveform group consisting of a plurality of pulse waveforms to obtain a data, and transmit the data to the computer via the interface;
wherein the blocking size m is equal to or more than a lower limit determined according to a size of the antigen or the antibody to be detected and is equal to or less than 1/2 of the pore diameter D, and the diameter d of the antibody-modified particle or the antigen-modified particle is 1/5 or more of the pore diameter D and less than the pore diameter D.

9. The device according to claim 8, wherein the blocking size m is equal to or less than 1/10 of the pore diameter D.

10. A program, comprising computer-readable instructions configured to be executed by a processor in a computer in which the processor is configured to be connected to a sensor via a network, the sensor comprising two chambers separated by a partition wall and communicating with each other through a pore, and an electrode in each of the two chambers; the computer readable instructions configured to execute the processor to perform:

a step of filtering a biological sample through a filter having a blocking size m to prepare a filtered sample;
a step of prepare a measurement target sample by mixing:

antibody-modified particles having a particle diameter d and an antibody that binds to the antigen attached to its surface, or antigen-modified particles having a particle diameter d and an antigen that binds to the antibody attached to its surface,
the filtered sample, and
a first electrolyte solution;

to a sensor having a structure in which two chambers separated by a partition wall having a pore with a pore diameter D communicate with each other through the pore, and an electrode is provided in each of the two chambers, a step of filling one of the two chambers of the sensor with the measurement target sample;
a step of filling the other of the two chambers of the sensor with a second electrolyte solution to electrically connect the two chambers through the pores;
a step of applying a voltage between two electrodes in each of the two chambers to allow an ionic current to flow between the two electrodes via the pore, and measuring a transient change in the ionic current that occurs each time the antibody-modified particle or the antigen-modified particle passes through the pore as a pulse waveform group consisting of a plurality of pulse waveforms; and
a step of estimating the presence or absence or the concentration of the antigen or the antibody to be detected in the biological sample by analyzing the pulse waveform group;
wherein the blocking size m is equal to or larger than a lower limit determined according to a size of the antigen or the antibody to be detected and is equal to or less than 1/2 of the pore diameter D, and the diameter d of the antibody-modified particle or the antigen-modified particle is 1/5 or more of the pore diameter D and less than the pore diameter D.

(A) Sensor structure

FIG. 1A

(B) Electron microscope
image of pore portion

FIG. 1B

FIG. 2

START

| | |
|---|---|
| A target antibody that specifically binds to a target antigen is attached to the surface of a detection particle to prepare an antibody-modified particle. | S301 |
| A biological sample with a known concentration of a target antigen is pretreated to prepare a known measurement target sample (described in FIG. 11). | S302 |
| The known measurement target sample is filled in one chamber of the sensor and a second electrolyte is filled in the other chamber. | S303 |
| A voltage is applied between the electrodes of the sensor, and the transient change in current when a particle passes through is measured as a pulse waveform. | S304 |
| The correlation between the presence or absence and concentration of a known target antigen and the shape and distribution of the pulse signal of a known measurement target sample is modeled (trained in the case of AI) to determine the agglutination-accelerating antigen concentration. | S305 |

END

(A) Modeling with known samples (training in the case of AI)

FIG. 3A

START

A target antibody that specifically binds to a target antigen is attached to the surface of a detection particle to prepare an antibody-modified particle. — S311

A biological sample with a known concentration of a target antigen is pretreated to prepare a known measurement target sample (described in FIG. 11). — S312

The measurement target sample is filled in one chamber of the sensor and a second electrolyte is filled in the other chamber. — S313

A voltage is applied between the electrodes of the sensor, and the transient change in current when a particle passes through is measured as a pulse waveform. — S314

The presence or absence and concentration of the target antigen in a test sample is estimated from the shape of the pulse signal, its distribution, and a correlation model (in the case of AI, a trained AI model). — S315

END

(B) Detection or quantification of unknown samples (in the case of AI, estimation by trained AI)

FIG. 3B

```
                    ┌─────────────┐
                    │    START    │
                    └─────────────┘
                           │
┌──────────────────────────────────────────────────────────────┐
│ After spinning down the particles for measurement, remove     │     S401
│ the supernatant and suspend the particles in the modification │
│ reaction buffer.                                              │
└──────────────────────────────────────────────────────────────┘
                           │
┌──────────────────────────────────────────────────────────────┐
│ Add any amount of antibody and mix overnight.                 │     S402
└──────────────────────────────────────────────────────────────┘
                           │
┌──────────────────────────────────────────────────────────────┐
│ After spinning down, remove the supernatant and modify it     │     S403
│ with bovine serum albumin.                                    │
└──────────────────────────────────────────────────────────────┘
                           │
┌──────────────────────────────────────────────────────────────┐
│ Wash the particles with bovine serum albumin solution buffer. │     S404
└──────────────────────────────────────────────────────────────┘
                           │
┌──────────────────────────────────────────────────────────────┐
│ Suspend particles in storage buffer.                          │     S405
└──────────────────────────────────────────────────────────────┘
                           │
                    ┌─────────────┐
                    │     END     │
                    └─────────────┘
```

FIG. 4

FIG. 5

FIG. 6

FIG. 7

FIG. 8

(A) Conventional method

FIG. 9A

(B) Method of present invention

FIG. 9B

FIG. 10

From S301, S311

| | |
|---|---|
| (In the case of microorganisms) Dilute the biological sample with a first electrolyte solution (extraction solution). | S1111 |
| Shake for antigen extraction incubation. | S1112 |
| Filtration through a filter with a blocking size of 1/10 or less of the pore diameter. | S1113 |
| Mix the filtered sample with the storage solution containing the antibody-modified particles. | S1114 |
| Shake for antigen-antibody reaction incubation | S115 |

S302
S312

To S303, S313

FIG. 11

Untreated culture supernatant — 1201

Antibody-modified particles — 1202

Antibody-modified particles + Untreated culture supernatant — 1203

Measurement target sample not according to present invention

1211

Treated supernatant

1209

Antibody-modified particles + Treated culture supernatant — 1210

Measurement target sample according to present invention

□ Measurement target particle

▨ Contaminant particles

0    50    100    150    200

Pulse number per 10 minutes    1200

FIG. 12

(A) Histogram of peak current per PCT copy

FIG. 13A

$$\sigma_b = \sqrt{\frac{1}{n} \sum_{i=1}^{n} (b_i - \mu)}$$

(B) Peak current standard deviation per PCT copy

FIG. 13B

(A) Particles passing

(B) Pulse waveform

An example in which D = 305 nm, $d_2$ = 220 nm, $m_2$ = 100 nm

FIG. 14

FIG. 15

## INTERNATIONAL SEARCH REPORT

| | International application No. |
|---|---|
| | **PCT/JP2023/016945** |

**A. CLASSIFICATION OF SUBJECT MATTER**

**G01N 27/00**(2006.01)i; **G01N 33/483**(2006.01)i; **G01N 33/543**(2006.01)i
FI:    G01N27/00 Z; G01N33/543 521; G01N33/483 E

According to International Patent Classification (IPC) or to both national classification and IPC

**B. FIELDS SEARCHED**

Minimum documentation searched (classification system followed by classification symbols)

G01N27/00-G01N27/10; G01N27/14-G01N27/24; G01N33/483; G01N33/543; C12M1/34; G01N15/12

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2022-059154 A (HITACHI, LTD.) 13 April 2022 (2022-04-13) | 1-3, 6-10 |
| | paragraphs [0001], [0013]-[0043], [0050]-[0053], [0060], fig. 1-5 | |
| A | | 4-5 |
| A | JP 2014-173936 A (TOSHIBA CORP.) 22 September 2014 (2014-09-22) | 1-10 |
| | entire text | |
| A | WO 2018/199179 A1 (NATIONAL UNIVERSITY CORPORATION TOKYO MEDICAL AND DENTAL UNIVERSITY) 01 November 2018 (2018-11-01) | 1-10 |
| | entire text | |
| A | WO 2021/239912 A1 (IMPERIAL COLLEGE INNOVATIONS LTD.) 02 December 2021 (2021-12-02) | 1-10 |
| | entire text | |
| A | TAKAKURA et al., Single-molecule detection of proteins with antigen-antibody interaction using resistive-pulse sensing of submicron latex particles, Applied Physics Letters, 21 March 2016, vol. 108, pp. 123701-1 to 123701-5 | 1-10 |
| | entire text | |

☑ Further documents are listed in the continuation of Box C.    ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **26 May 2023** | **06 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| :--- |
| **PCT/JP2023/016945** |

**C. DOCUMENTS CONSIDERED TO BE RELEVANT**

| Category* | Citation of document, with indication, where appropriate, of the relevant passages | Relevant to claim No. |
| :---: | :--- | :---: |
| P, A | BioJapan2022 バイオ×AI(5) 直野 典彦 氏, YouTube [online] [video], 25 October 2022, [retrieved on 24 May 2023], Internet : <URL : https://youtu.be/qmjwoZQAlrE> non-official translation (NAONO, Norihiko. Bio x AI (5)) 2:58, 7:34 | 1-10 |

Form PCT/ISA/210 (second sheet) (January 2015)

**INTERNATIONAL SEARCH REPORT**
Information on patent family members

International application No.

**PCT/JP2023/016945**

| Patent document cited in search report | | | Publication date (day/month/year) | Patent family member(s) | | Publication date (day/month/year) |
|---|---|---|---|---|---|---|
| JP | 2022-059154 | A | 13 April 2022 | (Family: none) | | |
| JP | 2014-173936 | A | 22 September 2014 | US | 2014/0255911 A1 | |
| WO | 2018/199179 | A1 | 01 November 2018 | US | 2020/0166506 A1 | |
| WO | 2021/239912 | A1 | 02 December 2021 | (Family: none) | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8236574 B
- US 9726636 B

- JP 6985687 B

**Non-patent literature cited in the description**

- **YUSKO, ERIK C. et al.** Real-time shape approximation and fingerprinting of single proteins using a nanopore.. *Nature nanotechnology*, 2017, vol. 12 (4), 360-367